# EUROPEAN PATENT SPECIFICATION

(11) **EP 2 010 903 B1**
(45) Date of publication and mention of the grant of the patent: **02.05.2012**
(21) Application number: 06819957.9
(22) Date of filing: 13.12.2006
(51) Int. Cl.: G01N 33/49, G01N 33/86

(54) **APPARATUS AND METHOD FOR THE DIAGNOSIS, PROGNOSIS AND PHARMACOLOGICAL MONITORING OF THE THROMBOTIC-ISCHEMIC AND HEMORRHAGIC PATHOLOGY OF THE CARDIOVASCULAR APPARATUS**
VORRICHTUNG UND VERFAHREN ZUR DIAGNOSE, PROGNOSE UND PHARMAKOLOGISCHER ÜBERWACHUNG DES THROMBOTISCH-ISCHÄMISCHEN UND HÄMORRHAGISCHEN KRANKHEITSBILDS DES HERZ-KREISLAUF-APPARATS
APPAREIL ET PROCEDE DESTINES AUX DIAGNOSTIC, PRONOSTIC ET SURVEILLANCE PHARMACOLOGIQUE DE LA PATHOLOGIE THROMBOTIQUE-ISCHEMIQUE ET HEMORRAGIQUE DE L'APPAREIL CARDIO-VASCULAIRE

(30) Priority: 14.12.2005 IT DA20050212 U
(43) Date of publication of application: 07.01.2009
(73) Proprietor: Sedicidodici Srl, 33170 Pordenone (IT); Centro di Riferimento Oncologico, I-33081 Aviano (IT)
(72) Inventor: DE MARCO, Luigino, I-33085 Maniago (IT); STEFFAN, Agostino, I-31015 Conegliano (IT); MAZZUCATO, Mario, I-30023 Concordia Sagittaria (IT)
(74) Representative: Petraz, Gilberto Luigi
(86) International application number: PCT/EP2006/069689
(87) International publication number: WO 2007/068727

(56) References cited:
- WO-A-93/00989
- WO-A-2006/066008
- VAN BREUGEL H H ET AL: "Effects of flow pulsatility on platelet adhesion to subendothelium." ARTERIOSCLEROSIS (DALLAS, TEX.) 1988 MAY-JUN, vol. 8, no. 3, May 1988 (1988-05), pages 332-335, XP008074273 ISSN: 0276-5047
- USAMI S ET AL: "DESIGN AND CONSTRUCTION OF A LINEAR SHEAR STRESS FLOW CHAMBER" ANNALS OF BIOMEDICAL ENGINEERING, PERGAMON PRESS, OXFORD, GB, vol. 21, no. 1, January 1993 (1993-01), pages 77-83, XP001028270 ISSN: 0090-6964
- MACHIN M ET AL: "Characterization of platelet adhesion under flow using microscopic image sequence analysis." THE INTERNATIONAL JOURNAL OF ARTIFICIAL ORGANS JUL 2005, vol. 28, no. 7, July 2005 (2005-07), pages 678-685, XP008074138 ISSN: 0391-3988

## Description

### FIELD OF THE INVENTION

The present invention concerns an apparatus and method for the diagnosis, prognosis and pharmacological monitoring of the thrombotic-ischemic and hemorrhagic pathology of the cardiovascular apparatus. To be more exact, the present invention allows the study "in vitro", in a closed circuit, of primary hemostasis and the formation of the coagulum.

### BACKGROUND OF THE INVENTION

Hemostasis is the process that leads to the stoppage of blood loss from damaged blood vessels and is essential for life. It can be divided into three main processes:
- adhesion and activation of the platelets;
- formation of fibrin;
- vascular contraction.

Thrombosis is an unwanted formation of a hemostatic blockage or a thrombus inside a blood vessel or the heart.

A pathological hemostatic event, whether thrombotic, due to the sudden lack of blood flow in a blood vessel, as in the case of coronaries in myocardial infarction, or hemorrhagic, represents a severe problem for the cardiovascular system. On the other hand, one of the most frequent manifestations of disease in the "Western world" is certainly associated with acquired cardiovascular alterations, such as atherosclerotic manifestations, aneurisms, athero-venous shunts, vasculitis, anomalies of the cardiac valves, atrial fibrillations and conduction diseases, thrombosis of the veins and arteries, and numerous genetic anomalies associated with hemostatic defects. These pathological manifestations can be present even in subjects which in their normal everyday life have never shown any pathological events and who, on the contrary, on the occasion of surgical operations, can incur serious hemorrhagic manifestations. It is recognized that cardiovascular disease represents the cause of death for 39% of men and 49% of women (Ital Heart J 2004; 5 (Suppl 3): 22S-37S).

In the last decade the number of genetic anomalies found in these hereditary or acquired defects has increased drastically.

In many cases, however, identifying the genetic anomalies does not lead to the conclusion that that particular patient will certainly have a pathological manifestation, just as on the other hand subjects with thrombotic or hemorrhagic manifestations do not always have a manifest genetic anomaly.

This situation, clearly difficult from a diagnostic and prognostic viewpoint, is due to the fact that the hemostatic system, which mainly controls the state of tissue repair, is characterized by a multi-factor combination of biochemical-biophysical actions to control and regulate the process, with an abundance of "redundant mechanisms", also able to compensate for possible non-serious deficiencies.

This makes it extremely important to have, in the clinical environment, an "ex vivo" functional test which can identify potential thrombotic and hemorrhagic risks in "silent" subjects for pathological cardiovascular manifestations.

As is known, the platelets are the main cellular protagonists of hemostatic processes and with their intrinsic adhesive and aggregative capacities normally control the repair of tissue damage, deriving from physiological events or following pathological insults.

In particular, the interaction between platelets and the vasal endothelium is important.

In a normal, undamaged blood vessel the endothelium performs an active role in preventing the formation of the thrombus. In fact, the endothelium generates and releases not only prostacyclin and nitroxide, which inhibit platelet aggregation, but also the activator of plasminogen and anti-coagulant substances such as heparin and heparin sulfate.

When an atherosclerotic plate is broken or a blood vessel is cut or damaged, the endothelium is damaged and the platelets adhere to the substances that make up the sub-endothelium, for example collagen, and are activated.

This adhesion implies the interaction between the sub-endothelial components like the Von Willerbrand factor, and the glycoproteic receptor complex GPIb-IX-V located on the surface membrane of the platelets.

The activation of the platelets leads to their changing shape, as they become flattened and pseudopodia are formed.

The mass of aggregated platelets forms a stopper which, together with the vasal constriction, maintains the hemostasis in the small vessels until the platelet stopper is reinforced by the fibrin.

However, in some conditions, the platelets can exasperate the process of repair, and are activated in an inappropriate manner if there is a pathological change in the blood vessels, for example atherosclerosis which can cause a dramatic event which leads to the occlusion of the blood vessel: thrombosis. This last event, if it occurs in "noble" districts such as the circulation of the brain or heart, can have fatal consequences.

To understand the mechanisms which lead to the formation and growth of an occlusive "mural thrombus" it is necessary to define the properties, which are variable, of the blood flow and the vascular wall. These variables, like the nature of the reactive surfaces, blood cells and vascular wall and the hydro-dynamic forces, can directly modulate the process of adhesion, activation, aggregation and growth of the platelet thrombi.

The primary phase of the "thrombotic cascade" implies that the platelets, first in movement with the blood flow, become irreversibly attached, stable adhesion, to the point of vascular damage, resisting the contrary action of the hydro-dynamic forces which tend to return them into the fluid (Bell GI, Science. 1978; 200(4342): 618-27).

The hydro-dynamic forces which oppose the adhesion increase as the shear rates increase, and are effectively contrasted by the action of the cytoadhesive substance Von Willerbrand Factor (vWF) (Savage, B et at al.. 1996; Cell 84: 289-297). Due to its own intrinsic properties the vWF is able to change conformational state once bonded to the collagen, the basic substance of the extra-cellular matrix, and subjected to high shear rates. In fact, in these conditions the vWF passes from a globular form, proper to its state in solution in the blood, to the elongated form, characterized by a fundamental increase in the adhesion sites available for the platelets (Siedlecky CA. et al.. Blood. 1996; 88(8): 2939-50). The vWF therefore increases its adhesive capacity with the increase in intensity of the flow; this peculiarity, associated with the capacity of the collagen to bind the platelets at low flow, causes the vWF-collagen association to represent a dynamic adhesion mechanism for the platelets, capable of adapting to any fluid-dynamic condition, of the vein or artery, physiological in the repair of the tissue damage or pathological such as for example in the breakage of the atherosclerotic plate (Kher N. et al. Semin Thromb Hemost. 2004; 30(6): 665-72). Other substances, like the proteoglycans, fibronectin, fibrinogen and other substances deriving from the endothelial cells, concur to improve the effectiveness of this adhesive model, adapting it to the various vascular districts and the different stimulations able to start the hemostatic process (Ruggeri ZM. J Thromb Hemost. 2003; 1(7): 1335-42).

It is also known that a hemorrhage or thrombosis can easily remain undiagnosed with the use of traditional analytical systems, such as the evaluation of the hemorrhage time, turbidimetric, potentiometric platelet aggregation, in luminescence or other.

In the diagnostic field of hemostatic pathologies, three apparatuses in particular are known, which will now be briefly illustrated (Harrison P. Blood Reviews. 2005; 19: 111-123).

The known apparatus PFA-100 (Dade-Behring) simulates platelet function at high shear rates. This known apparatus needs citrated blood to prevent coagulation taking place; however, this prevents normal platelet functioning, at least with regard to adhesion. The known apparatus PFA-100 also needs an external activator, ADP or Adrenalin. This known apparatus seems able to replace hemorrhage time, but is penalized by the high hematocrit, low platelet count and, in short, it is an instrument which is not very flexible and not sensitive to pre-thrombosis conditions.

The IMPACT (Diamed) apparatus is also known, which exploits the principle of the cone and plate analyzer (CPA) using whole blood and a polystyrene surface; this known apparatus measures the platelet aggregation at high shear rates and the adhesion to the surface of the plate; this known apparatus is sensitive to deficiencies in vWF and fibrinogen, but is not very sensitive to other proteins of the extra-cellular matrix.

The Ultegra-RPFA (Accumetrix) apparatus is also known, which consists of a turbidimetric aggregometer; however, it is only a technological evolution of the already-known aggregometric approach and therefore suffers from the same, extensive limitations.

The patent documents WO-A-93/00989, WO-A-94/16326 and WO-A-2006/066008 are also known, which concern perfusion chambers for analyzing thrombogenesis phenomena. In these known documents, the blood is taken in through a circuit and through the perfusion chamber by means of a vacuum generation pump, at least in the initial stages of the analysis. However, the vacuum generated substantially constitutes a variable expansion phase which acts with a force applied on an incompressible phase, that is, the blood. This prevents any control on the flow forces, making it impossible to calculate the vascular environment to which the analysis refers. Moreover, an application in conditions of microgravity is rendered impossible because the presence of one gaseous phase, even if rarefied, in the system inevitably entails a negative interference of the force of gravity, with the effect that emulsions are generated, which are totally incompatible with the physiological vascular environment that is to be simulated.

DE-A-1-031-3284 is also known, which describes the application of a pulsating pump for a cyclical application to a bi-reactor in which alternately nutritive substances are inserted and catabolic substances are removed from the cells being cultivated. Therefore, this application too is unsuitable for the physiological vascular environment that is to be simulated.

The following scientific publications are also known: BARSTAD R. M. et al: "A perfusion chamber developed to investigate thrombus formation and shear profiles in flowing native human blood at the apex of well-defined stenoses" ARTERIOSCLEROSIS AND THROMBOSIS: A JOURNAL OF VASCULAR BILOGY / AMERICAN HEART ASSOCIATION. DEC. 1994, vol. 14, no. 12, December 1994 (1994-12) pages 1984-1991 and DIAZ-RICART M. et al: "Digital image analysis of platelet-extracellular matrix interactions: studies in von Willerbrand disease patients and aspirin-treated donors." HAEMOSTASIS. 1994 JUL-AUG vol. 24, no. 4, July 1994 (1994-07), pages 219-229.

In these known publications a peristaltic pump is used to achieve perfusion. However, this type of pump is mechanically traumatic for the blood cells, which are vital and react to physical stress. The use of a pulsating pump in a closed circuit is known from van Brengel et al., Arteriosclerosis, vol. 8 (1988), pages 332-335.

A purpose of the present invention is to achieve an apparatus and method for the diagnosis, prognosis and pharmacological monitoring of the thrombotic-ischemic and hemorrhagic pathology of the cardiovascular apparatus which allows to analyze the hemostatic phenomenon in its entirety and complexity: platelet adhesion to the sub-endothelial matrix, activation, aggregation, growth of the platelet thrombus and its stability, under variable fluid-dynamic conditions according to the vascular environment considered.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the relative dependent claims describe other characteristics of the invention or variants to the main inventive idea.

In accordance with the above purpose, according to the present invention, an apparatus for the diagnosis, prognosis and pharmacological monitoring of the thrombotic-ischemic and hemorrhagic pathology of the cardiovascular apparatus comprises a system of tubes, in a closed circuit, in which the whole blood which is to be subjected to diagnosis is able to flow; the system of tubes faithfully reproduces the human blood circuit, and advantageously the actions of anti-thrombosis or anti-hemorrhage drugs are able to be analyzed therein.

The system of tubes comprises a chamber having a plurality of micro-channels, which, thanks to its geometric characteristics, effectively simulates the fluid-dynamic conditions of the blood in a capillary.

In this chamber, also called perfusion chamber or cartridge, that is, where liquids pass or flow, in this case blood taken from a human body, the biological phenomena occur of adhesion, activation, aggregation and growth of the platelet thrombus and its stabilization.

According to one feature of the present invention, inside the micro-channels of the perfusion chamber there is a cytoadhesive substrate, to simulate a damaged vessel surface.

The blood is made to flow in the tubes, and in particular in the micro-channels of the perfusion chamber, by pumping means, advantageously suitable to reproduce at least a pulsating flow, with blood pressures inside the closed circuit in physiological conditions.

The pulsating type pumping means and the closed circuit are able to simulate faithfully the human cardiovascular circuit and, in particular, are able to reproduce, according to the frequency, the pulsating coronary flow or the continuous flow of the small arteries and capillaries, under controlled fluid-dynamic conditions. The pulsating type pumping means prevents the presence of gas in the closed circuit, and eliminates the risk of production of emulsion, which invalidates the analysis.

In a preferential embodiment, the invention provides to detect the pressure of the blood in the circuit and in the perfusion chamber by means of suitable pressure measuring means, so as to control the fluid-dynamics of the circuit. The pressure measuring means controls the functioning of the pumping means so that, when the vasal constriction increases; caused by the growth of the thrombus, the flow of blood is maintained substantially constant, as happens with real physiological compensatory mechanisms.

The closed circuit achieved is also advantageous because it allows to eliminate the risk of leakages of blood from the apparatus, thus drastically reducing the risk of contamination to the operators, and increasing the overall conditions of cleanliness and hygiene.

According to the present invention, the hemostatic phenomena which lead to the formation of the platelet thrombus are monitored, in the perfusion chamber, by acquisition of data, for example images, by an optical acquisition means, which advantageously comprises at least a microscope, serially connected to filming means, for example a camera.

The filming means is for example, but not only, of the CCD type, and has an integrated recording/acquisition system, connected to electronic processing means, such as a digital computerized system which has a memorized program.

The program, when executed in the memory of the computerized system, is able to analyze in real time the morphological, kinetic and biochemical variations inside and outside the platelets and the formation of the thrombus and its stabilization, in response to different shear rates applied and different cytoadhesive surfaces.

According to an advantageous feature of the present invention, the apparatus is able to monitor the process of formation of the thrombus and furthermore allows to prevent the thrombotic event and consequent infarction.

Advantageously, in fact, the invention allows to recreate artificially and faithfully the vascular environment of the human body, allowing to monitor and analyze the possible formation of thrombi "step by step".

Advantageously, the formation of the thrombus in the micro-vessels, that is, their constriction, is not the only event that can be detected by the invention since, thanks to its teaching, it is possible to detect both the steps before the formation of the thrombus, that is, the first adhesive steps of the platelets on the suitable surface, the platelet activation step, and also the subsequent step when the thrombus is formed, that is, the constriction of the blood vessel, and its possible consolidation and dissolution. In particular, the invention is sensitive to specific inter-platelet biochemical events, on individual platelets, aggregates or thrombi, such as intra-cellular calcium or other substance characteristic of activation processes, irrespective of the activation process. This is possible because the invention provides a real-time detection of the analytical data, thanks to a continuous detection and display during analysis, unlike in the state of the art, at a numerical or image frequency for detecting the analytical data which is equal to or higher than the average capacity of the human eye, that is, equal to or higher than 25 images per second. Lower frequencies, like an image every 10 seconds, as in WO-A-2006066008, make the detection system insensitive to phenomena of platelet activation, which occur in a time range of some hundredths of a second.

Furthermore, the present invention allows to perform the diagnostic test in different clinical conditions, for example in the frequent conditions of platelet deficiency or high hematocrit.

Advantageously, moreover, the present invention is also used in monitoring the effectiveness of the replacement therapy with platelet concentrates in different pathologies, oncological and otherwise.

Another advantageous feature of the invention is that the perfusion chamber is made from a single plastic block in which micro-vessels are made using laser-based techniques; the micro-vessels have a diameter comprised between 50 and 100 micron, and therefore the coating of the necessary cyto-adhesive occurs over all the surface of the vessel. This is considerably different, for example, from WO-A-93/00989, where the perfusion chamber is provided with a so-called "cover slip", on which the coating is exclusively made.

Moreover, unlike the BARSTAD publication, the perfusion chamber made here is not provided with initially stenotic channels. In fact, the presence of stenosis would entail the formation of flow perturbations, of the vortex or chaotic flow type, and this would impede the fluid-dynamic control of the circuit and of the perfusion chamber. Moreover, the formation of a perturbed flow in the circuit of the present invention can be detected by means of a linear TV camera, and analyzed as a numerical matrix, in order to describe a feature of the thrombus formation.

Advantageously, therefore, the perfusion chamber can be disposable, that is, used and then discarded, ready for use and part of a closed system, totally automated, in order to perform a complete test in a short period of time and using only one chamber per exam.

Advantageously, the plastic used, for example Cole Olefin Copolymer or COC, or other optically inert plastic, in order to achieve the perfusion chambers, is transparent to light radiation, is inert with regard to UV rays, is not auto-fluorescent and has a high capacity to bind proteins, that is, more than 60%.

An innovative feature of the present invention is the capacity to perform the analyses in conditions of absolute hydraulic seal, since the circuit and the perfusion chamber determine as a whole a hydraulically closed system throughout the analytical process. This ensures that there is no gaseous contamination in any part of the circuit, also thanks to the fact that sampling test tubes are used, containing the anti-coagulant substances and the markers used, and liquid containers of the apparatus, including for discharge, consisting of sacks of soft plastic material, that is, compressible, provided with non-return valves. During sampling, the entrance to the sack which functions as a sampling test tube consists of a normal sampling set with a needle and a protection valve, while the relative outlet consists of a manual suction syringe. When the sack that functions as test tube is full of anti-coagulated blood, without gas inside it, it is ready for automatic loading into the analysis apparatus.

This embodiment with its hydraulic seal allows to perform the analysis, in an absolutely innovative manner, also in conditions of micro-gravity, or in any case gravity unlike the Earth's gravity, and can also operate on installations or vehicles not on Earth, for example to analyze and monitor the formation of thrombi in astronauts.

### BRIEF DESCRIPTION OF THE DRAWINGS

These and other characteristics of the present invention will become apparent from the following description of a preferential form of embodiment, given as a non-restrictive example with reference to the attached drawings wherein:
- fig. 1 is a diagram of an apparatus according to the present invention;
- fig. 2 is an image of the adhesion of the platelet to the cytoadhesive substrate, in this specific case a versican;
- fig. 3 is another image of the adhesion of the platelet to the versican;
- fig. 4 is a graph of the development over time of the adhesion of the platelet to the versican;
- fig. 5 is a diagram of the functioning of a platelet receptor system;
- fig. 6 is a schematic view of a part of the apparatus in fig. 1;
- fig. 7 is a schematic view of a constructional variant of the part in fig. 6;
- fig. 8 is another schematic view of the part in fig. 7;
- fig. 9 is a diagram of the operating sequence of the part in fig. 6 or 7;
- fig. 10 is a schematic view of a pressure sensor of the apparatus in fig. 1;
- fig. 11 is a graph of the development of the pressure of the blood flow in the apparatus in fig. 1;
- fig. 12 shows a sequence of images of the interaction of platelets with the surface of the substrate and analysis of the plastic support material of the apparatus in fig. 1;
- fig. 13 is a schematic view of a part of the apparatus in fig. 1;
- fig. 14 is a graph of the development of the blood pressure in the apparatus in fig. 1;
- fig. 15 is a graph of the profile of the speed of the blood in the apparatus in fig. 1;
- fig. 16 is a schematic perspective view of a part of the apparatus in fig. 1;
- fig. 17 is a lateral view of the part of fig. 16; and
- fig. 18 is an image of the formation of a platelet thrombus in the apparatus in fig. 1.

### DETAILED DESCRIPTION OF A PREFERENTIAL FORM OF EMBODIMENT

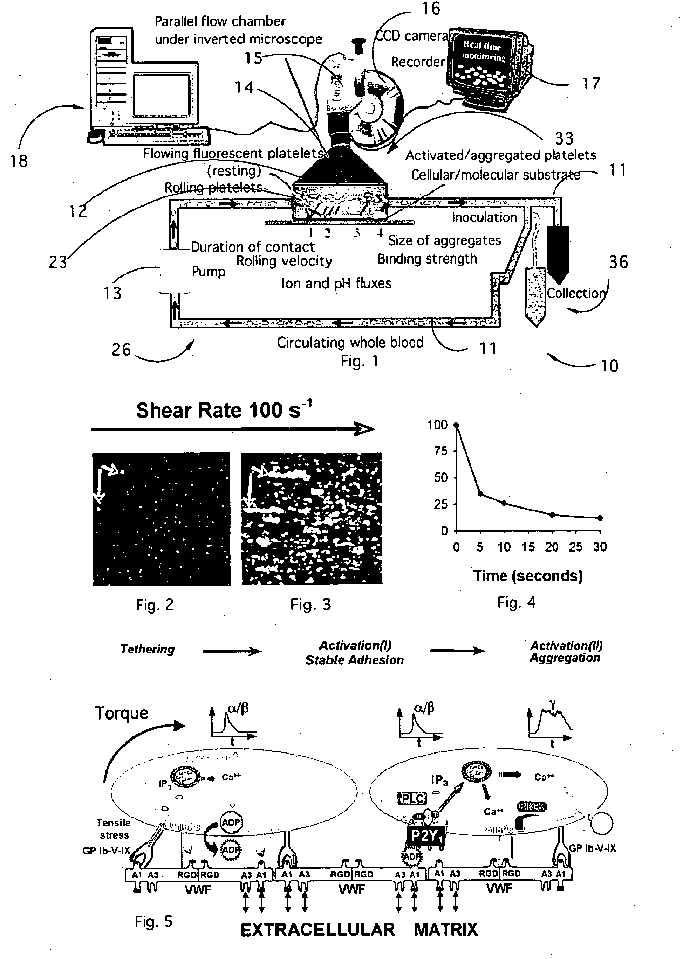

In accordance with the present invention, fig. 1 shows with the reference number 10 an apparatus for the diagnosis, prognosis and pharmacological monitoring of the thrombotic-ischemic and hemorrhagic pathology of the cardiovascular apparatus.

In the course of experiments done, the Applicant has defined numerous parameters which concern the main investigative characteristics of an effective diagnostic test, capable of discerning putative thrombotic or hemorrhagic anomalies in healthy subjects but which can derive from genetic and/or environmental predispositions. The diagnostic apparatus and method according to the present invention must necessarily involve platelet functionality because, as shown above, it represents the main protagonist of the hemostatic process.

However, the other cellular blood components, leucocytes, erythrocytes, micro-particles, and soluble blood components, coagulative factors, ionic composition, can play decisive roles.

For this reason Applicant has chosen to use, in the present invention, "whole blood", not manipulated and sufficiently anti-coagulated, by means of PPACK, Irudina or others, so as not to interfere with the adhesive and activating mechanisms of the blood cells.

Since the morphological feature of platelet adhesion and aggregation is closely associated with the intra-cellular activation events that involve signal transduction phenomena, it is of fundamental importance to coordinate these morphological evaluations with intra-cellular biochemical variations which the platelets have, and to monitor them in real time.

To meet these requirements, according to the present invention, the apparatus 10 comprises a completely closed and sealed circuit 26, comprising tubular elements 11 in which the blood flows, with collection and discard elements 36, able to be selectively connected to the closed circuit 26 by means of valves not shown in the drawings, a computerized image analysis system, comprising optical acquisition means 33 and electronic processors 17, 18, which is based on the use of fluorescent probes introduced inside the platelets and re-suspended in whole blood, a perfusion chamber 12 where the phenomena of adhesion-activation-aggregation-formation of the platelet thrombus and its stabilization are made to occur, and a sophisticated software able to follow in real time and at high frequency (at least 25 images per second) the morphological, kinetic and biochemical variations inside the platelets (Ca++, NO, Ph...).

According to the present invention, the electronic processor 17 is able to monitor in real time the flow conditions in the perfusion chamber 12, while the electronic processor 18 is able to perform the computerized analysis of the platelet functioning, "platelet storm".

According to the present invention, the experimental plan developed by Applicant provides to use a perfusion chamber 12 for example with a parallel flow (figs. 1, 16, 17) where the hemostatic phenomena occur which lead to the formation of the platelet thrombus.

The processes which occur in the chamber 12 are monitored and analyzed by a digital computerized system comprising the electronic processors 17, 18 by means of a software able to analyze the formation of the thrombus in response to different shear rates applied to different cytoadhesive surfaces.

The apparatus according to the present invention is equipped with fluidically interconnected tubular elements in which the blood is able to flow and suitable to achieve a closed circuit 26 which simulates a hematic circuit, in which the actions of anti-thrombosis or anti-hemorrhage drugs can also be analyzed.

According to one form of embodiment of the present invention, the flow of blood is controlled by pumping means 13, such as an intake pump-syringe or pulsating pump, and the images are acquired by optical acquisition means 33 which comprise at least a microscope 14 and a video acquisition device 15, for example a CCD-camera, advantageously associated with recording means 16.

In this case, by microscope 14 we mean an opto-electronic system consisting of a poly-monochromatic illuminator, also a laser, an autofocus lens, which cooperates with a matrix or linear or detector type TV camera 15, which allow a microscopic type vision.

The images are derived from the microscope 14 and are captured by the combination of the CCD-camera 15 and the recorder 16, which can advantageously be digital or analogical. The platelets can be marked with a suitable fluorescent probe according to the acquisition apparatus and to the biological phenomenon to be monitored, for example mepacrin or fluo3-AM in the case of a fluorescent microscope being used, with fluo4-AM if a confocal microscope or other is used.

Alternately, instead of a system able to acquire images, it is also possible to use other systems of an optical type, which use for example a led laser device with semiconductors, like a laser velocimeter.

The analytical capacities of the apparatus 10 are such as to detect the dynamics of the cell contact with the adhesive surfaces, and between cells, the duration of the relative bindings, the spatial distribution of the aggregates, the dynamics of the growth of the platelet thrombus and, by studying the movements of the intra-cytoplasmic calcium, to study the transmission of the activating signals inside the cells.

Advantageously, the present invention has been developed by adapting this technology to easy and versatile conditions of use, and is suitable for simple routine analyses or, in a different configuration, for more sophisticated studies for basic and pharmacological research.

According to the present invention, the experimentation was directed to identifying the ideal cytoadhesive substrate, which must be present inside the perfusion chamber 12 to simulate a damaged vessel surface, to be used both at low and high shear rates.

This feature is particularly important because it allows, advantageously by varying only the physical feature of the flow rate, to simulate the venous and/or arterial environment. The results of the experimental analysis of various components of the extra-cellular matrix are summarized in Table 1. Table 1 reports various cytoadhesive substances present inside the perfusion chamber 12 used as a prototype according to the present invention, to study the adhesive capacity and various platelet parameters.

**Table 1**

| Cytoadhesive substrate | Platelet | | | |
|---|---|---|---|---|
| | Frequency of adhesion | Duration of contact | Motility | Aggregation |
| Collagen type I/III | ++ | ++ | -- | ++ |
| Collagen type VI | ++ | ++ | -- | ++ |
| PG-M/versican (vascular) | ++ | + | + | (+) |
| Perlecan Fibronectin | -- | -- | -- | -- |
| Laminin-1 (-2, -4, -5, -8, -10, -11) | -- | -- | -- | -- |
| Vitronectin | -- | -- | -- | -- |
| Decorin | + | + | + | -- |
| Biglycan | + | + | + | -- |
| Fibulin-1 (-2) | -- | -- | -- | -- |
| Tenascin-C | -- | -- | -- | -- |
| Lumican | -- | -- | -- | -- |
| | -- | -- | -- | -- |
| Trombospondin-1 | -- | -- | -- | -- |

From Table 1, and in accordance with previous publications, the collagens and the von Willerbrand factor are able to promote adhesion and aggregation of the platelets. As shown in Table 1, this series of experiments demonstrates the capacity of some PG-M/versicans, proteoglycanic substances of the extra-cellular matrix, to produce a certain stable adhesion of the platelets and which are also able to considerably increase, once mixed with the collagens, the aggregative capacity and the growth of the platelet thrombus (Mazzucato M et al. FASEB J. 2002; 16(14): 1903-16), although they are not able to promote platelet activation. Advantageously, as can be seen from Table 1, the substance used effectively as a cytoadhesive substrate is collagen type I.

Figs. 2 and 3 show images in real time of the number of platelets which form transitory contacts with the substrate Versican V1/V2 deriving from the aorta sub-endothelium. The images are captured by the optical acquisition means 33 and recorded by the image recording means 16, such as a video-recorder, at the speed of 25 images/sec. (PAL signal) during a period of 30 sec. and summed one over the other in layers. On the left, fig. 2, the first image captured appears, with two leucocytes indicated by the arrows. Fig. 3 shows the sum of the images, where two leucocytes can be seen (arrows) which have performed a travel during the 30 sec. of the duration of the recording. The average speed is about 2.67 ± 0.13 µm/s and around 15% of the adherent platelets transfer onto the Versican surface V1/V2 with an average speed of about 1.05 ± 0.1 µm/s, while the remaining platelets show a stable temporary adhesion.

Fig. 4 shows the diagram of the percentage of platelets which have stopped in the same position for the time in seconds indicated on the x axis.

To obtain detailed information on the processes of platelet activation during the adhesion and aggregation phase inside the perfusion chamber 12 of the present invention, which appears of fundamental importance for the biological and clinical interpretation of the observations, Applicant analyzed the fluxes of intra-platelet calcium (Ca++), the main local messenger of the transduction of the intra-cytoplasmic signal using the vWF (von Willerbrand Factor) as a cytoadhesive substrate.

Using an innovative computerized analysis system, associated with specific software developed by Applicant, by means of the invention it was discovered that the interaction of the platelets with the vWF, in conditions of high shear rates, produces distinct increases in cytosolic Ca++ which are correlated to the state of activation.

The tension stress, the mechanical action between the glyco-proteic platelet receptor GPIbα and the domain A1 of the vWF leads to a rapid and transitory release of Ca++ by the intra-cytoplasmic deposits (α/β type peaks), which precede the stable adhesion of the platelets.

Stable adhesion is in turn characterized by the binding of the platelet integrin αIIbβ3 to the RGD sequence of the vWF and this binding event entails an increase in intra-cytoplasmic Ca++ which is more considerable and lasting over time and characterized by a flux of Ca++ arriving from outside (γ type peaks) (Mazzucato M. et al. Blood. 2002; 100(8): 2793-800). Then, a signal which originates in the stretching of the mechanoreceptor GPIbα irreversibly bonded with the vWF (domain A1) leads to a sufficient activation of the integrin αIIbβ3, to allow a localized adhesion.

An additional signal arriving from the ADP released by the platelet, bonded with its receptors P2Y1 and P2Y12, is essential for total activation and for the growth of the platelet thrombus (fig. 3). Fig. 5, in fact, shows a schematic representation of the role of the platelet receptor of the ADP, P2Y1, on the platelet activation induced by the vWF, immobilized on the surface, and in conditions of high shear rates (300 s-1). The domain A1 of the vWF interacts with the platelet GPIbα (Savage B. Cell. 1998 Sep 4;94(5): 657-66), whereas the RGD sequence in the domain Cl of the vWF interacts with the activated integrin αIIbβ3. In fig. 5, by PIP2 we mean phosphatidyl-inositol-4,5-triphosphate, by IP3 we mean inositol-1,4,5-triphosphate, by DG we mean diacylglycerol, by PLC we mean phospholipase C, by PKC we mean protein kinase C and by PI 3-K we mean phosphatidylinositol 3-kinase. ADP is shown to interact with one of its two receptors coupled with the G protein and characterized by seven trans-membrane domains: P2Y1, associated with Ca++ released by the internal deposits, and with PLCβ. The position of the peaks of [Ca2+] ᵢα/β is relative to the engagement of the GPIb-IX-V of the domain A1 of the vWF and the amplification signal through the ADP-P2Y1 interaction; the peak γ represents the signal evoked by the bonding of the domain Cl of the vWF to the integrin αIIbβ3 and activation of the PI 3-K (Mazzucato et al. Blood. 2004 Nov 15;104(10):3221-7).

By means of the experiments illustrated above, and other, similar ones, Applicant identified a series of five different groups of substances or materials which can be used as a cytoadhesive substance in the perfusion chamber according to the invention, to simulate the vessel lesion. The different experimental conditions can potentially be used for different clinical purposes.

The groups of substances or materials that can be used as a cytoadhesive substrate substance, with reference to Table 1, are:
- a group A which comprises substrate substances which lead to the adhesion, activation, aggregation and formation of the platelet thrombus with high shear rates, like those found in arteries and capillaries, including the vWF;
- a group B which comprises substrate substances which act at low shear rates, like those found in veins, fibrinogen, collagens;
- a group C which comprises substate substances which support platelet adhesion but not activation and aggregation and only at low shear rates, fibronectin, PGM/versicans and perlecan;
- a group D which comprises substrate substances which potentiate the pro-thrombotic activity of other molecules, additive effects at low and/or high shear rates, such as certain proteo-glycans, fibulins involved in the platelet adhesion, and fibrinogen;
- a group E which comprises substrate substances which act as anti-thrombotic substances at various shear rates, such as various proteo-glycans, hyaluronic acid and certain glyco-proteins.

Moreover, the simulation of the perfusion system and optronic analysis by means of the perfusion chamber 12 and the optical acquisition means 33 which cooperate with the electronic processing means 17, 18 proved itself to be a valid choice for the development of the subsequent technological phase. To be more exact, the application of the constant laminar flow in the perfusion chamber 12 proved itself to be extremely valid in identifying coagulative defects of the hemorrhagic and also thrombotic type, as shown in confirming data already published in subjects subjected to physical stress (Rock G. et al. Clin J Sport Med. 1997; 7(2): 94-9).

According to the present invention, the means 13 for pumping the blood comprises a pulsating pump 19, 20 suitable to reproduce a discontinuous flow of blood inside the closed circuit 26. In this way, a fluid-dynamic component is introduced into the system which makes the flow in the perfusion chamber 12 very similar to the conditions present in the coronary flow, faithfully reproducing what really happens in the circuit that irrorates the heart. The pulsating pump means 13 can be selectively set so as to apply a constant flow, if the analysis conditions require. In other words, the invention is embodied to function with a constant flow or with a pulsating flow, so as to advantageously adapt to every circulation condition, advantageously having a great versatility of use.

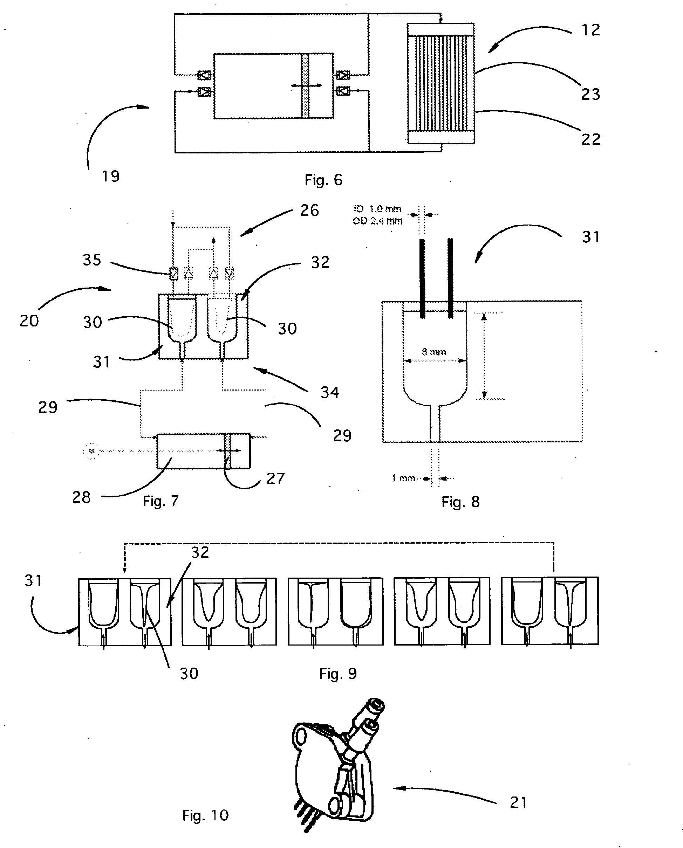

According to the present invention, the use of the pulsating pump 19 allows to reproduce a pulsating flow, unidirectional through perfusion tubes 23 or channels of the cartridge 12, 22 by means of two variable volume closures, as represented schematically in fig. 6.

Advantageously, the flow is directed through a unidirectional valve. The operation of the pump 19 is very similar to a human heart, because both systems use pulsating cavities with variable volume, pseudo-ventricles according to the present invention, and unidirectional valves.

Advantageously, the pumping means 13, according to one embodiment of the present invention as shown in fig. 7, separates the mechanical generation of the pressure and intake from the circuit containing the blood and in fig. 7 the relative coupled circuits can be seen.

According to the present invention, the pressure cycle is generated by a piston 27 in alternate movement in a steel chamber 28, moved by an eccentric wheel of a step-by-step motor. The control of the speed of rotation and of the eccentric wheel allows to determine the frequency of pulsation and the amplitude of pressure. The pressure inside the air circuit 29 is transmitted to the closed circuit 26 through a latex membrane 30 (shown by a line of dashes in fig. 7), which expands or contracts in conformity with the air pressure present around it. This conformation is particularly suitable for use with small quantities of blood. Both the pseudo-ventricles 31, 32 are contained in a single block 34 of transparent material, such as Perspex, which allows to see the functioning of the membrane 30.

The operating cycle is schematized summarily in fig. 9. It is based on an alternation of pressurization and depressurization of the two pseudo-ventricles 31, 32 so that the latex membranes 30 or pockets can fill and empty. The pneumatic piston 27 alternates depressurization and pressurization on each pseudo-ventricle 31, 32, as shown in fig. 9.

According to an advantageous form of embodiment of the present invention, each pseudo-ventricle 31, 32, where the latex membrane 30 separates the blood from the air, possesses the sizes indicated in fig. 8. The maximum volume of each pseudo-ventricle 31, 32 is approximately about 350 µl. According to the present invention, the volume available for maximum expansion is not necessarily completely filled, in fact the maximum volume at the end of each cycle depends on the length of travel of the piston 27 in the cylinder.

Since the pseudo-ventricles 31, 32 are critical components in the context of the present invention, Applicant studied and developed them on purpose.

In particular, the pseudo-ventricles 31, 32 comprise unidirectional valves 35 which have been obtained by means of spheres, made of biocompatible plastic material, free to move between two parts in a ring, one constructed so as to have a uniform contact with the sphere, contact with which determines a stoppage of the blood flow, the other constructed so as to keep the sphere in suspension so that the flow passes around it. A specific software, which takes into account the times-pressure deriving from a flow transducer, regulates the speed of the piston 27. In this way it is possible to control effectively a pressure profile that can simulate the physiological or pathological profile. The whole microcirculation system is kept at a constant temperature of about 37°C, controlled by a microprocessor which guarantees errors in the order of ±0.5°C.

The measurements of pressure are obtained by means of the pressure measurement sensors 21, such as a differential pressure transducer, for example Motorola MPX5100, in contact with the blood flow. The series of pressure sensors 21 Motorola MPX is based on a silicon-based piezoelectric mechanism, which allows a connection with the blood circuit 26 by means of a minimum-length T junction; according to the present invention, the sensors 21 are positioned upstream and downstream of the perfusion chamber 12, to assess the fall in pressure in the latter. The sensors 21 supply an outlet signal to the microprocessor approximately between 0.2 Vdc at 0 mmHg It and at 375 mmHg of applied pressure. It is directly connected with the data acquisition system embodied by the processors 17, 18, so as to be used to monitor the formation of the vessel obstruction in the perfusion chamber 12 and simultaneously to control the frequency and pressure applied to the closed circuit 26.

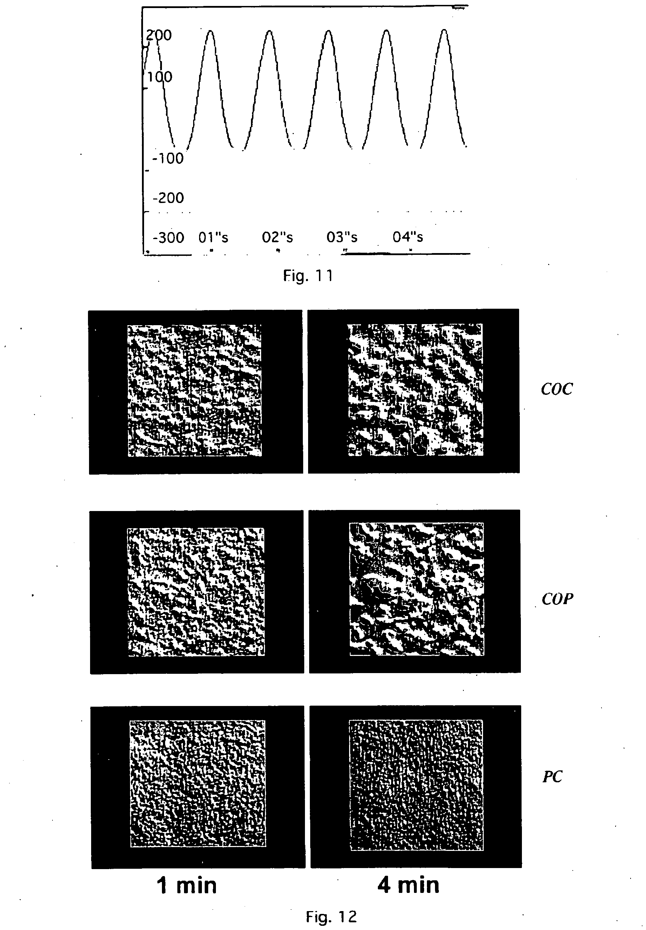

The acquisition software determines the sampling fraction and the number of readings per unit of time. A sampling frequency of more than 50 Hz (50 cycles/sec) can easily be obtained and is easily sufficient for monitoring the pressure variations which cyclically represent the systolic and diastolic pressure of cardiac rhythm. The pressure values are acquired through a digital acquisition card and managed by means of a PC with a standard operating system of a known type. For example, in this application, the card uses 8 analogical input channels and two output channels, together with 16 digital I/O connections. The A/D conversion is effected by the same card, advantageously with a 12 bit resolution, in which the entire period of the signal is resolved in 4096 sub-divisions. The acquisition software allows to store long observation intervals and executes numerous numerical analyses in order to identify pathological pressure behaviors or to record the progression of the growth of the platelet thrombus. The software can also monitor the energy consumption of the motor of the pump 19, 20, which can supply further information on the reduction in the gap of the perfusion chamber 12 due to the growth of the platelet thrombus. Fig. 11 shows a typical recording of pressure over time using the sensor 21 and the pump 20 as described above.

The pumping means 13 allows to reproduce a pulsating flow, with blood pressures inside the circuit in physiological conditions. Moreover, the pulsating pump 19, 20 according to the present invention allows to easily manipulate small quantities of blood.

According to another advantageous feature, the blood is exposed to biocompatible materials, in order to prevent contamination and activation and at the same time to supply an optimum support for the cytoadhesive substrates and be suitable for optical detections; to this end, moreover, the pumping means 13 thus embodied allow to prevent external contaminations, with great safety for the operator.

We also have optimum adaptability of the perfusion chamber 12, or cartridge 22, to different shear rates with a maximum shear rate ≥ 1500 s-1 and limited mechanical stress to the blood and its cellular components.

For the construction of the perfusion chamber 12 of the apparatus according to the invention, Applicant studied numerous and different plastic materials to test their characteristics of biocompatibility and adaptability to the analytical conditions suggested by the experimental investigations conducted with the video-image apparatuses described above. For the comparative analyses, Applicant chose type I collagen as substrate, due to its excellent cytoadhesive capacities. The studies were carried out using the constant and parallel flow system as described above. As plastic support material for the type I collagen, Applicant used COC (cyclic copolymers with an olefinic base), COP (cyclic polymers with an olefinic base), conventional polystyrene, polycarbonate and glass as a control support. These materials generically satisfy the requirements of homogeneous thickness, compatibility of the visual field with the high-resolution image analysis, fluorescent microscopy and real time acquisition. Another fundamental characteristic is the great capacity for binding the cytoadhesive substances to be used as the substrate for platelet adhesion.

Applicant then carried out perfusion experiments with platelets marked with mepacrin and the results indicate that no satisfactory platelet adhesion with polycarbonate (PC) was detected, irrespective of the type of cytoadhesive substance used as substrate. Advantageously, the adhesive substrates immobilized on COC and COP show platelet adhesion and aggregation. The formation of the platelet thrombus on the COC incubated with collagen type I and III was found satisfactory, like the formation using the vWF, with a rapid growth kinetic and stability at all shear rates used. In fact, the height of the thrombus in about 4 minutes exceeds 100 µm. Therefore, the choice of plastic material for the construction of the perfusion cartridge advantageously falls on COC. In fact, fig. 12 shows a real time observation, after about 1 min and about 4 min of perfusion, of the interaction of the platelets with the collagen I surface, disposed on different plastic materials. The type I collagen, with a concentration of about 100 µg/ml in PBS, was applied to COC, COP and polycarbonate. The substrate was assessed in a Hele-Show type parallel flow chamber, modified to produce variable flows. The blood was anti-coagulated with PPACK, treated with the fluorescent probe mepacrin and then perfused through the chamber 12 at the temperature of 37°C; in this case, the total area of the optical field corresponds to 0.037 mm².

Based on previous acquisitions, a multi-channel perfusion chamber or cartridge 22 was made, that is, comprising a plurality of tubes 23 or micro-channels. The choice of multi-channels was advantageously dictated by the need to have on the one hand multiple measurements so as to be able to obtain statistically correct and significant values of the phenomenon being observed, and on the other hand due to the possible need to simultaneously use several cytoadhesive substances and therefore have qualitatively different responses for the same blood sample. This last observation can be extremely advantageous from the point of view of using a very limited quantity of sample.

An important factor, studied by Applicant, in the construction of the cartridge made of plastic material (COC) concerns the mechanism of the fluids inside the micro-channels 23. To study this feature Applicant chose a bio-engineering approach. The characterization of the dynamics of the blood flow in the micro-channels 23 has two important features: one concerning the whole cartridge 22, the other concerning the surface inside the micro-channels 23 where platelet adhesion and the growth of the thrombus take place. For the first feature, the planning and design of the entire unit is important, where a quantitative relationship between the fraction of flow and the fall in pressure must be found, and at the same time a uniform distribution of the flow along the parallel micro-channels 23 guaranteed. The reproduction of a uniform flow line inside the micro-channels 23 concerns a local dimension of the analysis of the blood flow. The simulation of the mechanics of the fluid allows to reconstruct the precise distribution of the local hemodynamic parameters, such as pressure, speed of flow and shear rate, which are crucial in determining the mechanisms of platelet adhesion and activation. These simulations serve to correctly design the cartridge 22 with micro-channels or capillaries 23 of the invention. The aligned capillaries are fed by a common tube 37, relatively wide with respect to the inlet of the micro-channels 23, which must provide the uniform distribution of the fluid and reduce the conditions of disturbed flow inside the capillaries 23. During experimentation different forms of tubes and connections with the capillaries 23 were simulated.

The local speed and distribution of pressures of the blood flow through the capillaries 23 were determined advantageously by means of a numerical solution of the Navier-Stokes equation, concerning the conservation of moment and mass. Applicant considered that the blood fluid possesses constant density and viscosity, thus obtaining detailed predictions on the components of speed, pressure and shear rate, in the current geometry through the use of the software CFX-4.4 (AEA Technology, UK), highly efficient and confirmed in numerous technical and scientific applications which concern the study of fluid mechanics in complex geometries. For these applications it was assumed that blood is a homogeneous fluid, chemically inert, incompressible and with a Newtonian behavior; surprisingly, considering the behavior of a "granular" fluid like blood as Newtonian was experimentally demonstrated as a valid assumption for shear rate values above 100 s-1, values which affect the experimentation relating to the invention. Moreover, the blood fluid was assumed to have a laminar regimen, a condition which is certainly present inside the blood capillaries in physiological conditions. In all conditions the following values were adopted: 1056 Kg/m3 for density; 0.0035 Paxs for viscosity.

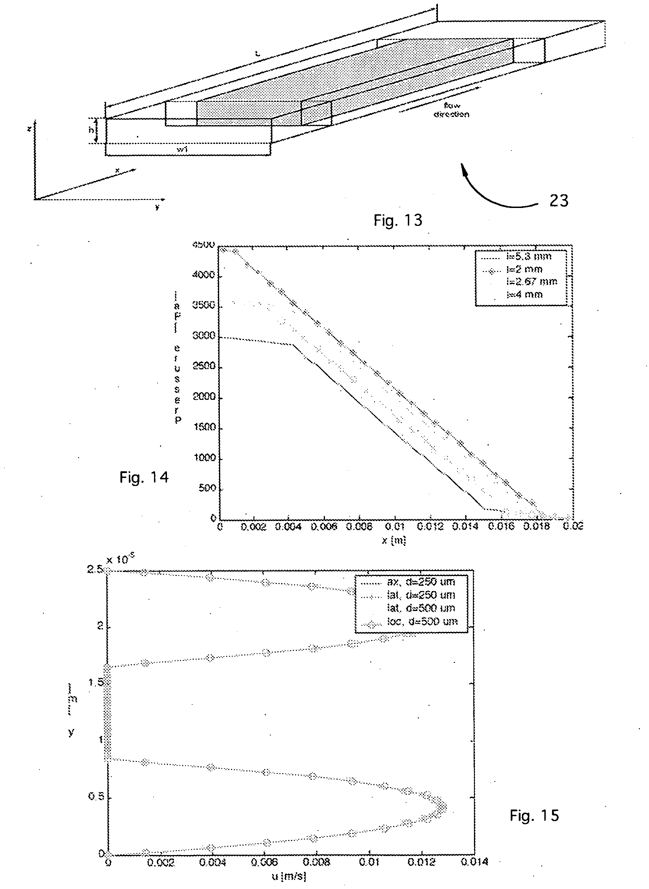

For the sake of simplicity, and to give an example, the simulation of only one capillary 23 is reported here, representative of all those provided for a cartridge. The capillaries 23 were advantageously made with a square section, with a size per side of 50 µm. The simplified multi-channel model is shown in fig. 13. It consists of two capillaries positioned opposite a border area, an INLET zone which represents the distributor-connector to ensure a total development of the speed profile, an OUTLET zone with the same volume as the distributor-connector. The distance between two capillaries 23 is a multiple of the width of a capillary 23. The height of the system corresponds to the height of the capillary 23 and the length is arbitrarily fixed at 15 mm, but can be variable as desired and to meet contingent requirements. Subsequently, the length was verified to be long enough to allow a parabolic profile of the speed of the fluid inside the capillaries. The total width is 0.35 mm. The blood flow was determined so as to normally produce a shear rate at the wall of the capillary 23 of about 1500 s⁻¹.

The computational grid was defined for the capillaries 23, in particular for their terminal parts, so as to detect in an accurate and realistic manner the motion of the fluid in these regions. Different solutions were simulated for the inlet of the fluid, starting from the most simplified, such as for example the centered solution in line with the channels.

The results of copious simulation calculations can be summarized by observing that:
- the drop in pressure through the cartridge 22 is dominated by resistance along the capillaries 23. This can be seen in fig. 14, which reports the development of the pressure along the cartridge 22 by varying the length of the distributor-connector 37 and where the simulation for different sizes of the distributors-connectors 37 is compared;
- the great drop in pressure in the capillaries 23 allows a uniform pressure in the distributor-connector 37, irrespective of the localization of the inlet;
- the speed profile of the blood in the capillaries 23 is totally developed in a few diameters, with an irrelevant fluid-dynamic disturbance given the length of about 400 diameters. Therefore, the speed profile assumes almost perfectly a parabolic profile, Poiseuille, as shown in fig. 15, where a comparison is shown between different inlet configurations of the blood fluid in terms of speed profile in the capillaries at a few diameters from their beginning.

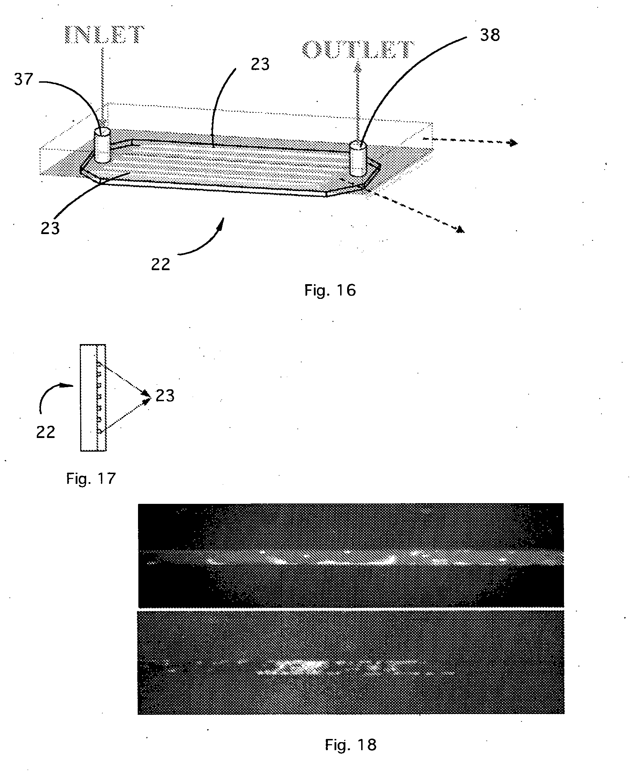

The cartridge 22 with micro-channels 23 was therefore designed according to all the fluid-dynamic simulations, as it appears in figs. 16, 17, and constructed of COC, as suggested by the functional tests illustrated. The cartridge 22 is connected to the closed circuit 26, in which there is the blood fluid, by means of HPLC-like tubes, to reduce the dead spaces, and consists of biocompatible materials, for example Tygon, Teflon or others.

Applicant conducted numerous preliminary tests with the present invention as described and the results confirm the effectiveness of the experimental plant adopted for the experimentation. In fact the blood made to flow in pulsating conditions inside the cartridge at the rate of 25 µl/min develops a shear rate of 1500 s-1, at the physiological pressure of 120/80 mmHg. Fig. 18 shows the micro-channels according to the present invention, in which the type I collagen was previously immobilized as cytoadhesive substance and which represent the formation of the platelet thrombus.

Then, whole human blood extracted from a human body was made to flow at 37°C, anti-coagulated with an anti-thrombotic substance (P-PACK). The platelets were marked with mepacrin 8 µM. The images show the formation of the platelet thrombus in two different moments. At 60 sec the capillary is completely obstructed and advantageously the whole growth of the thrombus is recorded in real time as described above.

Advantageously, by means of the present invention there was a compression of the molecular mechanisms of the formation of the thrombus.

Moreover, an advantageous system with a closed circuit 26 was achieved, which simulates the human blood circulation under controlled fluid-dynamic conditions.

Advantageously, the perfusion chamber 22 (cartridge) with micro-channels 23 is made as a disposable device.

According to the present invention, moreover, analytical methods were defined, identified when determining pressure, speed of flow, computerized image analysis for the study of hemodynamics.

All these advantageous features were explored and positively assessed during the experimental tests performed and reported above.

The choice of the anti-coagulant was also explored, given the importance of keeping integral the ionic environment in the present invention, preferably choosing an anti-thrombotic agent like Irudina or P-PACK. Advantageously, moreover, the test can be correctly performed within two hours from the blood sample being taken from the human body and the execution time of the test can be fixed in about three minutes. Advantageously, the expectations for the functioning follow the results already obtained and widely reported in literature. The prototype is advantageously assembled in the two foreseen configurations, one for a "lighter" screening and equipped with a system to detect pressure and electric consumption, the other intended for second-level diagnosis and for research, equipped with optronic systems implemented with image analysis, echo-Doppler or others.

## Claims

1. Apparatus for the diagnosis, prognosis and pharmacological monitoring of the thrombotic-ischemic and hemorrhagic pathology of the cardiovascular apparatus, **characterized in that** it comprises:
- a plurality of tubular elements (11) fluidically connected to achieve a closed circuit (26) in which a sample of blood is able to flow;
- pumping means (13) of the pulsating type able to pump the blood in said closed circuit (26) in controlled and variable fluid-dynamic conditions;
- at least a perfusion chamber (12), comprising a multi-channel perfusion cartridge (22) formed by a plurality of micro-channels (23) in fluidic connection with said closed circuit (26) and in which the blood is able to flow, said micro-channels (23) being able to simulate fluid-dynamic conditions of the blood analogous to the fluid-dynamic conditions of blood flowing in the human body so that phenomena and processes of hemostasis are reproducible, said circuit (26) and perfusion chamber (12) determining as a whole a hydraulically closed system throughout the analytical process;
- optical acquisition means (33), suitable to acquire, by means of a real-time and continuous detection, data at high frequency on the blood circulating in the micro-channels (23) of said cartridge (22);
- electronic processing means (17, 18), suitable to process at least said data acquired by said optical acquisition means (33), to selectively effect said diagnosis, prognosis and pharmacological monitoring.

2. Apparatus as in claim 1, **characterized in that** the micro-channels (23) are capillaries having a diameter comprised between 50 and 100 micrometer.

3. Apparatus as in claim 1 or 2, **characterized in that** the micro-channels (23) are fed by a common tube (37), relative wide with respect to the inlet of the micro-channels (23) so as to guarantee the uniform distribution of the fluid and reduce the conditions of disturbed flow inside said micro-channels (23).

4. Apparatus as in claim 1, 2 or 3, **characterized in that** said micro-channels (23) are disposed parallel to each other.

5. Apparatus as in any claim hereinbefore, **characterized in that** said optical acquisition means (33) comprises a microscope (14) serially connected to a video acquisition device (15).

6. Apparatus as in any claim hereinbefore, **characterized in that** it comprises recording means (16) suitable to record said data acquired by said optical acquisition means (33) and to render said recorded data available to said electronic processing means (17, 18).

7. Apparatus as in any claim hereinbefore, **characterized in that** said pumping means (13) of the pulsating type comprises a pulsating pump (19, 20) able to reproduce a pulsating flow analogous to the pulsating flow of the human heart.

8. Apparatus as in any claim hereinbefore, **characterized in that** it comprises pressure measuring means (21) able to detect pressure data at least in said tube (23) of said perfusion chamber (12), and able to send said pressure data to said electronic processing means (17, 18), so that they can be analyzed and processed.

9. Apparatus as in any claim hereinbefore, **characterized in that** said micro-channels (23) are made of a polymeric material.

10. Apparatus as in claim 9, **characterized in that** said polymeric material is a plastic material and is chosen from a group comprising: polycarbonate, cyclical copolymers with an olefinic base and cyclical polymers with an olefinic base.

11. Apparatus as in any claim hereinbefore, **characterized in that**, inside the micro-channels (23) of said cartridge (22), a cytoadhesive substrate substance is provided which is able to simulate a damaged vessel surface.

12. Apparatus as in claim 11, **characterized in that** said cytoadhesive substrate substance is comprised in said tube (23).

13. Apparatus as in claim 11 or 12, **characterized in that** said cytoadhesive substrate substance is chosen from a group comprising:
- substances in group A comprising substrate substances which lead to the adhesion, activation, aggregation and formation of the platelet thrombus with high shear rates, like those found in arteries and capillaries, including the vWF;
- substances in group B comprising substrate substances which act at low shear rates, like those found in veins, fibrinogen, collagens;
- substances in group C comprising substrate substances which support platelet adhesion but not activation and aggregation and only at low shear rates, fibronectin, PGM/versicans and perlecan;
- substances in group D comprising substrate substances which potentiate the pro-thrombotic activity of other molecules, additive effects at low and/or high shear rates, such as certain proteo-glycans, fibulins involved in the platelet adhesion, and fibrinogen;
- substances in group E comprising substrate substances which act as anti-thrombotic substances at various shear rates, such as various proteo-glycans, hyaluronic acid and certain glyco-proteins.

14. Method for the diagnosis, prognosis and pharmacological monitoring of the thrombotic-ischemic and hemorrhagic pathology of the cardiovascular apparatus, **characterized in that** it comprises the steps of:
- making a closed circuit (26) available, in which a sample of blood is able to flow;
- making pumping means (13) of the pulsating type available, able to pump the blood in said circuit;
- making a perfusion chamber (12) available, comprising a multi-channel perfusion cartridge (22) formed by a plurality of micro-channels (23) in fluidic connection with said closed circuit (26), said micro-channels (23)being able to simulate fluid-dynamic conditions of the blood analogous to the fluid-dynamic conditions of blood flowing in the human body and in order to simulate the hemodynamics of the human blood, said circuit (26) and perfusion chamber (12) determining as a whole a hydraulically closed system throughout the analytical process;
- making the blood flow inside said closed circuit (26) by means of said pumping means (13) of the pulsating type;
- monitoring, by means of a real-time and continuous detection of data at high frequency on the blood circulating in the micro-channels (23) of said cartridge (22), the development of the flow of blood at least in the micro-channels (23) of said cartridge (22) in order to selectively effect said diagnosis, prognosis and pharmacological monitoring of the thrombotic-ischemic and hemorrhagic pathology of the cardiovascular apparatus.

15. Method as in claim 14, **characterized in that** it comprises the steps of:
- making optical acquisition means (14) available, suitable to acquire data on the flow of blood circulating at least in the micro-channels (23) of the cartridge (22));
- making electronic processing means (17, 18) available, suitable to receive said data acquired by said optical acquisition means (14);
- effecting said monitoring step by means of said optical acquisition means (14) and said electronic processing means (17, 18).

## Patentansprüche

1. Vorrichtung für die Diagnose, Voraussage und die pharmakologische Überwachung der thrombotisch-ischämischen und hämorrhagischen Pathologie des Herzkreislauf-Apparats, **dadurch gekennzeichnet, daß** sie umfaßt:
- eine Vielzahl von Schlauchelementen (11), die strömungstechnisch zu einem geschlossenen Kreislauf (26) verbunden sind, in dem eine Blutprobe fließen kann;
- eine Pumpeinrichtung (13) des pulsierenden Typs, die in der Lage ist, das Blut unter gesteuerten und regelbaren strömungstechnischen Bedingungen in den genannten geschlossenen Kreislauf (26) zu pumpen;
- zumindest eine Perfusionskammer (12), umfassend eine Mehrkanal-Infusionskassette (22), gebildet durch eine Vielzahl von Mikrokanälen (23), die in strtimungstechnischer Verbindung zu dem genannten geschlossenen Kreislauf (26) stehen, und in der das Blut fließen kann, wobei die genannten Mikrokanäle (23) in der Lage sind, die strömungstechnischen Bedingungen des Blutes analog zu den strömungstechnischen Bedingungen des Blutflusses im menschlichen Körper zu simulieren, so daß Phänomene und Vorgänge der Hämostase reproduzierbar sind, und wobei der genannte Kreislauf (26) und die Perfusionskammer (12) als Ganzes ein hydraulisch geschlossenes System während des ganzen analytischen Verfahrens festlegen;
- eine optische Erfassungseinrichtung (33), die geeignet ist, Daten des in den Mikrokanälen (23) der Kassette (22) zirkulierenden Bluts mit hoher Frequenz in Echtzeit- und Dauerüberwachung zu erfassen;
- eine elektronische Verarbeitungseinrichtung (17, 18), die geeignet ist, zumindest die genannten, durch die optische Erfassungseinrichtung (33) erfassten Daten zu verarbeiten, um wahlweise die genannte Diagnose, Voraussage und pharmakologische Überwachung durchzuführen.

2. Vorrichtung nach Anspruch 1, **dadurch gekennzeichnet, daß** die Mikrokanäle (23) Kapillaren mit einem Durchmesser zwischen 50 und 100 Mikrometern sind.

3. Vorrichtung nach Anspruch 1 oder 2, **dadurch gekennzeichnet, daß** die Mikrokanäle (23) durch eine gemeinsame Leitung gespeist werden, die relativ groß ist im Vergleich zu der Einlaßöffnung der Mikrokanäle (23), um die gleichmäßige Verteilung der Flüssigkeit sicherzustellen und die Bedingungen für eine gestörte Strömung in den Mikrokanälen (23) zu verringern.

4. Vorrrichtung nach Anspruch 1, 2 oder 3, **dadurch gekennzeichnet, daß** die genannten Mikrokanäle (23) parallel zueinander angeordnet sind.

5. Vorrichtung nach einem der vorhergehenden Ansprüche, **dadurch gekennzeichnet, daß** die optische Erfassungseinrichtung (33) ein Mikroskop (14) umfaßt, das mit einem Video-Erfassungsgeriit (15) in Serie geschaltet ist,

6. Vorrichtung nach einem der vorhergehenden Ansprüche, **dadurch gekennzeichnet, daß** sie eine Aufnahmeeinrichtung (16) umfaßt, die geeignet ist, die genannten, durch die genannte optische Erfassungseinrichtung erfaßten Daten aufzunehmen und die aufgenommenen Daten für die genannte elektronische Verarbeitungseinrichtung (17, 18) verfügbar zu machen.

7. Vorrichtung nach einem der vorhergehenden Ansprüche, **dadurch gekennzeichnet, daß** die Pumpeinrichtung (13) des pulsierenden Typs eine Pulsationspumpe (19, 20) umfaßt, die geeignet ist, eine pulsierende Bewegung analog der pulsierenden Bewegung des menschlichen Herzens nachzwnachen.

8. Vorrichtung nach einem der vorhergehenden Ansprüche, **dadurch gekennzeichnet, daß** sie eine Druckmeßeinrichtung (21) umfaßt, die geeignet ist, Druckmeßwerte zumindest in den genannten Kanälen (23) der genannten Perfusionskammer (12) zu erfassen, und geeignet ist, die genannten Druckmeßwerte zu der genannten elektronischen Verarbeitungseinrichtung (17, 18) zu senden, so daß sie analysiert und verarbeitet werden können.

9. Vorrichtung nach einem der vorhergehenden Ansprüche, **dadurch gekennzeichnet, daß** die genannten Mikrokanäle aus polymerem Material hergestellt sind.

10. Vorrichtung nach Anspruch 9, **dadurch gekennzeichnet, daß** das genannte polymere Material ein Kunststoff ist und ausgewählt aus der Gruppe umfassend: Polycarbonat, cyclische Copolymere mit einer olefinischen Basis und cyclische Polymere mit einer olefinischen Basis.

11. Vorrichtung nach einem der vorhergehenden Ansprüche, **dadurch gekennzeichnet, daß** im Innern der Mikrokanäle (23) der Kassette (22) eine Substanz in Form eines cytoadhäsiven Substrats bereitgestellt ist, die geeignet ist, eine geschädigte Gefäßoberfläche zu simulieren.

12. Vorrichtung nach Anspruch 11, **dadurch gekennzeichnet, daß** die Substanz in Form des cytoadhäsiven Substrats in den Kanälen (23) enthalten ist.

13. Vorrichtung nach Anspruch 11 oder 12, **dadurch gekennzeichnet, daß** die Substanz in Form des cytoadhäsiven Substrats ausgewählt ist aus der Gruppe umfassend:
- Substanzen in Gruppe A, die Substratsubstanzen umfassen, welche zu Adhäsion, Aktivierung, Aggregation und Blutplättchen-Thrombenbildung mit hohen Scherraten führen, wie solche, die in Arterien und Kapillaren gefunden werden, einschließlich des vWF;
- Substanzen in Gruppe B, die Substratsubstanzen umfassen, welche bei geringen Scherraten wirken, wie die, welche in Venen, Fibrinogen und Collagenen zu finden sind;
- Substanzen in Gruppe C, die Substratsubstanzen umfassen, welche das Verkleben der Blutplättchen unterstützen, aber nicht die Aktivierung und Aggregation, und nur bei geringen Scherraten, Fibronectin, PGM/Versikan und Perlecan;
- Substanzen in Gruppe D, die Substratsubstanzen umfassen, welche die prothrombotische Aktivität anderer Moleküle verstärken, zusätzlichen Einfluß bei geringen und/oder hohen Scherraten, wie bestimmte Proteoglycane, an der Blutplättchen-Verklebung beteiligte Fibuline, und Fibrinogen;
- Substanzen in Gruppe E, die Substratsubstanzen umfassen, welche bei verschiedenen Scherraten als anti-thrombotische Substanzen wirken, wie verschiedene Proteoglycane, Hyaluronsäure und bestimmte Glycoproteine,

14. Verfahren zur Diagnose, Voraussage und pharmakologischer Überwachung der thrombotisch-ischämischen und hämorrhagischen Pathologie des Herzkreislauf-Apparats, **dadurch gekennzeichnet, daß** es die Schritte umfaßt:
- Bereitstellen eines geschlossenen Kreislaufs (26), in dem eine Blutprobe strömen kann;
- Bereitstellen einer Pumpeinrichtung (13) des pulsierenden Typs, die in der Lage ist, das Blut in den genannten Kreislauf zu pumpen;
- Bereitstellen einer Perfusionskammer (12), die eine Mehrkanal-Infusionskassette (22) umfaßt, gebildet durch eine Vielzahl von Mikrokanälen (23), die in strömungstechnischer Verbindung zu dem genannten geschlossenen Kreislauf (26) stehen, wobei die genannten Mikrokanäle (23) in der Lage sind, die strömungstechnischen Bedingungen des Blutes analog zu den strtimungstechnischen Bedingungen des Blutflusses im menschlichen Körper zu simulieren, um die Hämodynamik des menschlichen Blutes zu simulieren, und wobei der genannte Kreislauf (26) und die Perfusionskammer (12) als Ganzes ein hydraulisch geschlossenes System während des ganzen analytischen Verfahrens festlegen;
- Bilden des Blutflusses innerhalb des genannten geschlossenen Kreislaufs (26) durch die Pumpeinrichtung (13) des pulsierenden Typs;
- Überwachen der Entwicklung des Blutflusses zumindest in den Mikrokanälen (23) der genannten Kassette (22) durch eine Echtheit- und Dauererfassung der Blutzirkulation in den Mikrokanälen (23) der genannten Kassette (22) mit hoher Frequenz, um wahlweise die genannte Diagnose, Voraussage und die pharmakologische Überwachung der thrombotisch-ischämischen und hämorrhagischen Pathologie des Herzkreislauf-Apparats durchzuführen.

15. Verfahren nach Anspruch 14, **dadurch gekennzeichnet, daß** es die Schritte umfaßt:
- Bereitstellen einer optischen Erfassungseinrichtung (14), die geeignet ist, Daten über den zumindest durch die Mikrokanäle (23) der Kassette (22) zirkulierenden Blutfluß zu erlangen;
- Bereitstellen einer elektronischen Verarbeitungseinrichtung (17, 18), die geeignet ist, die genannten, durch die optische Erfassungseinrichtung (14) erfassten Daten aufzunehmen;
- Durchführen des genannten Überwachungsschritts durch die genannte optische Erfassungseinrichtung (14) und die genannte elektronischen Verarbeitungseinrichtung (17, 18).

## Revendications

1. Appareil pour le diagnostic, le pronostic et le suivi pharmacologique de la pathologie thrombotique-ischémique et hémorragique de l'appareil cardio-vasculaire, **caractérisé en ce qu'**il comprend :
- une pluralité d'éléments tubulaires (11) raccordés fluidiquement pour réaliser un circuit fermé (26) dans lequel un échantillon de sang peut circuler ;
- un moyen de pompage (13) de type pulsatoire pouvant pomper le sang dans ledit circuit fermé (26) dans les conditions fluide-dynamiques contrôlées et variables ;
- au moins une chambre de perfusion (12), comprenant une cartouche de perfusion multi-canaux (22) formée par une pluralité de micro-canaux (23), en liaison fluidique avec ledit circuit fermé (26) et dans lequel le sang peut circuler, lesdits micro-canaux (23) pouvant simuler les conditions fluido-dynamiques du sang, analogues à celles du sang circulant dans le corps humain, de manière à reproduire les phénomènes et processus d'hémostase, lesdits circuit (26) et chambre de perfusion (12) déterminant ensemble un système hydrauliquement fermé tout au long du processus analytique ;
- un moyen d'acquisition optique (33) destiné à acquérir, au moyen d'une détection continue et en temps réel, des données à haute fréquence sur la circulation sanguine dans les micros-canaux (23) de ladite cartouche (22) ;
- des moyens de traitement électronique (17, 18), destinés à traiter au moins lesdites données acquises par ledit moyen d'acquisition optique (33), pour effectuer sélectivement lesdits diagnostic, pronostic et suivi pharmacologique.

2. Appareil selon la revendication 1, **caractérisé en ce que** les micro-canaux (23) sont des capillaires d'un diamètre compris entre 50 et 100 micromètres.

3. Appareil selon la revendication 1 ou 2, **caractérisé en ce que** les micro-canaux (23) sont alimentés par un tube commun (37), relativement large par rapport à l'entrée des micro-canaux (23), de manière à garantir la distribution uniforme du fluide et à réduire les conditions de perturbation de l'écoulement à l'intérieur desdits micro-canaux (23).

4. Appareil selon la revendication 1, 2 ou 3, **caractérisé en ce que** les micro-canaux (23) sont disposés parallèlement les uns aux autres.

5. Appareil selon l'une quelconque des revendications précédentes, **caractérisé en ce que** le moyen d'acquisition optique (33) comprend un microscope (14) monté en série sur un dispositif d'acquisition vidéo (15).

6. Appareil selon l'une quelconque des revendications précédentes, **caractérisé en ce qu'**il comprend un moyen d'enregistrement (16) destiné à enregistrer lesdites données acquises par ledit moyen d'acquisition optique (33), et à mettre lesdites données enregistrées à la disposition desdits moyens de traitement électronique (17, 18).

7. Appareil selon l'une quelconque des revendications précédentes, **caractérisé en ce que** ledit moyen de pompage (13) de type pulsatoire comprend une pompe de pulsation (19, 20) pouvant reproduire un écoulement pulsatoire analogue à celui du coeur humain.

8. Appareil selon l'une quelconque des revendications précédentes, **caractérisé en ce qu'**il comprend un moyen de mesure de pression (21) pouvant détecter les données de pression au moins dans ledit tube (23) de ladite chambre de perfusion (12) et envoyer lesdites données de pression auxdits moyens de traitement électronique (17, 18), de manière à pouvoir à la fois les analyser et les traiter.

9. Appareil selon l'une quelconque des revendications précédentes, **caractérisé en ce que** lesdits micro-canaux (23) sont en matériau polymère.

10. Appareil selon la revendication 9, **caractérisé en ce que** ledit matériau polymère et un matériau plastique choisi dans un groupe comprenant :
le polycarbonate, les copolymères à base d'oléfine cyclique et les polymères à base d'oléfine cyclique.

11. Appareil selon l'une quelconque des revendications précédentes, **caractérisé en ce qu'**une substance de substrat cytoadhésif est prévue à l'intérieur des micro-canaux (23) de ladite cartouche (22), pour simuler une surface de vaisseau endommagé.

12. Appareil selon la revendication 11, **caractérisé en ce que** ladite substance de substrat cytoadhésif est comprise dans ledit tube (23).

13. Appareil selon la revendication 11 ou 12, **caractérisé en ce que** ladite substance de substrat cytoadhésif est choisie dans un groupe comprenant :
- des substances du groupe A comprenant des substances de substrat qui conduisent à l'adhésion, l'activation, l'agrégation et la formation du thrombus plaquettaire avec des taux de cisaillement élevés, comme ceux que l'on trouve dans les artères et capillaires, incluant le vWF ;
- des substances du groupe B comprenant des substances de substrat qui agissent à des taux de cisaillement faibles, comme ceux que l'on trouve dans les veines, le fibrinogène, les collagènes :
- des substances du groupe C comprenant des substances de substrat qui supportent l'adhésion plaquettaire, mais ni l'activation ni l'agrégation, et seulement à des taux de cisaillement faibles, la fibronectine, les PGM/versicans et le perlecan ;
- des substances du groupe D comprenant des substances de substrat qui favorisent l'activité prothrombotique d'autres molécules, les effets additifs à des taux de cisaillement faibles et/ou élevés, telles que certains protéoglycanes, les fibulines impliquées dans l'adhésion plaquettaire et le fibrinogène :
- des substances du groupe E comprenant des substances de substrat qui agissent comme substances anti-thrombotiques à différents taux de cisaillement, telles que les divers protéoglycanes, l'acide hyaluronique et certaines glyeoprotéines.

14. Procédé pour le diagnostic, le pronostic et le suivi pharmacologique de la pathologie thrombotique-ischémique et hémorragique de l'appareil cardio-vasculaire, **caractérisé en ce qu'**il comprend les étapes suivantes :
- mettre à disposition un circuit fermé (26), dans lequel un échantillon peut s'écouler ;
- mettre à disposition un moyen de pompage (13) de type pulsatoire, pouvant pomper le sang dans ledit circuit ;
- mettre à disposition une chambre de perfusion (12), comprenant une cartouche de perfusion multi-canaux (22) formée par une pluralité de micro-canaux (23) en liaison fluidique avec ledit circuit fermé (26), lesdits micro-canaux (23) pouvant simuler les conditions fluido-dynamiques du sang, analogues à celles du sang circulant dans le corps humain, de manière à simuler l'hémodynamique du sang humain, lesdits circuit (26) et chambre de perfusion (12) déterminant ensemble un système hydrauliquement fermé tout au long du processus analytique ;
- faire circuler le sang à l'intérieur dudit circuit fermé (26) au moyen dudit moyen de pompage (13) de type pulsatoire ;
- suivre l'évolution de l'écoulement de sang au moins dans les micro-canaux (23) de ladite cartouche (22), au moyen d'une détection de données continue et en temps réel à haute fréquence, sur la circulation sanguine dans les micro-canaux (23) de ladite cartouche (22), de manière à réaliser sélectivement lesdits diagnostic, pronostic et suivi pharmacologique de la pathologie thrombotique-ischémique et hémorragique de l'appareil cardio-vasculaire.

15. Procédé selon la revendication 14, **caractérisé en ce qu'**il comprend les étapes suivantes :
- mettre à disposition un moyen d'acquisition optique (14), destiné à acquérir des données sur la circulation sanguine au moins dans les micros-canaux (23) de la cartouche (22) ;
- mettre à disposition des moyens de traitement électronique (17, 18), destinés à recevoir lesdites données acquises par ledit moyen d'acquisition optique (14) ;
- effectuer ladite étape de suivi au moyen dudit moyen d'acquisition optique (14) et desdits moyens de traitement électronique (17, 18).
